# FASCICULE DE BREVET EUROPEEN

(11) **EP 1 261 387 B1**
(45) Date de publication et mention de la délivrance du brevet: **20.07.2005**
(21) Numéro de dépôt: 01909886.2
(22) Date de dépôt: 23.02.2001
(51) Int. Cl.: A61M 5/30, A61M 5/48, A61M 5/303

(54) **SERINGUE SANS AIGUILLE A DEUX NIVEAUX DE VITESSE D'INJECTION**
NADELLOSE SPRITZE MIT ZWEISTUFIGER INJEKTIONSGESCHWINDIGKEIT
NEEDLELESS SYRINGE WITH TWO INJECTION SPEED LEVELS

(30) Priorité: 01.03.2000 FR 0002633
(43) Date de publication de la demande: 04.12.2002
(73) Titulaire: Crossject, 75181 Paris Cédex 04 (FR)
(72) Inventeur: ALEXANDRE, Patrick, F-70100 Gray (FR); BROUQUIERES, Bernard, F-83000 Toulon (FR); GAUTIER, Philippe, F-91220 Le Plessis Pate (FR); ROLLER, Denis, F-91590 La Ferte Alais (FR)
(86) Numéro de dépôt international: PCT/FR2001/000536
(87) Numéro de publication internationale: WO 2001/064269

(56) Documents cités:
- DE-A- 3 405 671
- US-A- 2 704 543
- US-A- 3 605 744
- US-A- 4 447 225
- US-A- 5 520 639

## Description

La présente invention est dans le domaine des seringues sans aiguille utilisées pour les injections intradermiques, sous-cutanées ou intramusculaires de principe actif liquide à usage thérapeutique en médecine humaine ou vétérinaire. Le document US-A-5520639 représente l'état de la technique le plus proche et détermine le préambule de la revendication 1.

Par définition une seringue sans aiguille est non invasive : il n'y a pas d'aiguille qui traverse la peau pour amener le principe actif là où il doit agir. Pour une seringue sans aiguille, il faut que le jet de principe actif liquide sortant d'un orifice ou conduit d'injection perce la peau et pénètre plus ou moins profondément suivant le type d'injection souhaité : pour cela le jet doit avoir une grande vitesse. Si le jet est trop lent, il n'y a pas perforation de la peau, le liquide se répand à la surface de la peau et il est perdu car il ne produit pas d'effet thérapeutique.

Mais, une fois que le liquide a percé la peau et atteint la profondeur voulue, la vitesse du jet peut être plus faible pour assurer le passage et la diffusion dans la peau du principe actif liquide restant à injecter.

Pour assurer une bonne biodisponibilité du principe actif il est donc souhaitable d'avoir, en début d'injection, une vitesse élevée pour le jet de liquide puis une vitesse plus faible pour assurer le passage du liquide restant. Ces deux régimes de vitesse correspondant dans le liquide, à des pressions d'injection fortes puis plus faibles.

Le brevet US 2 704 543 traite de ce problème et propose pour le résoudre un moyen de poussée constitué par deux pistons concentriques agissant sur un obturateur déformable permettant l'expulsion du liquide. Le piston interne, de petite section, agit initialement seul sur l'obturateur déformable et produit une forte pression d'injection, puis les deux pistons concentriques sont déplacés simultanément afin de réduire la pression d'injection puisque la section efficace de poussée est augmentée alors que l'effort de poussée agissant sur les deux pistons reste la même que celui agissant sur un seul piston. Cet effort est produit par la détente d'un ressort comprimé.

Le brevet US 3 335 722 décrit une seringue sans aiguille avec un générateur de gaz agissant sur un moyen de poussée constitué par un piston unique, dont l'extrémité dirigée vers le principe actif est étagée. Ce piston agit sur un obturateur déformable permettant l'expulsion complète du liquide contenu dans le réservoir, la variation de pression d'injection est obtenue uniquement par le contrôle du débit de gaz généré et par l'augmentation du volume libre en amont du piston, la section efficace du piston de poussée étant constante.

Les seringues proposées dans ces brevets ne permettent pas de contrôler les deux paliers de pression, notamment la durée du palier de pression initiale est très difficilement réglable. Ces seringues utilisent de plus un obturateur déformable et de géométrie complexe pour réaliser l'expulsion complète du principe actif.

De plus ces seringues sont conçues pour être utilisées plusieurs fois et comportent plusieurs dispositifs annexes nécessaires au rechargement en principe actif et à la réactivation des moteurs.

Le but de la présente invention est de proposer des seringues simples, permettant de réaliser deux paliers de pression et de vitesse d'injection de niveau et de durée prédéterminés. Ces seringues pourraient être jetables après utilisation.

La présente invention concerne une seringue sans aiguille, pour l'injection d'un principe actif liquide, comme définie dans la revendication 1.

Le long de la course motrice la force agissant sur le moyen de poussée est la résultante de la pression s'exerçant sur la grande section de la tête motrice : la pression transmise au liquide est forte et donc la vitesse d'injection est élevée. Après cette course motrice, la tête motrice cesse d'être active, l'effort résultant sur le moyen de poussée est la résultante de la pression s'exerçant sur une section plus petite : celle du réservoir ; la pression dans le liquide est alors plus faible, de même que la vitesse d'injection du liquide.

Dans cette invention par principe actif liquide nous entendrons essentiellement un liquide plus ou moins visqueux, ou un mélange de liquides, ou un gel. Le principe actif pourra être un solide mis en solution dans un solvant approprié pour l'injection. Le principe actif pourra être un solide sous forme pulvérulente mis en suspension, plus ou moins concentrée, dans un liquide approprié. La granulométrie du principe actif solide doit être adaptée ainsi que la forme du conduit pour éviter les bouchages.

La course motrice efficace de la tête motrice est inférieure à la hauteur du réservoir de principe actif. Avantageusement cette course motrice efficace est inférieure à 0,6 fois la hauteur du réservoir et préférentiellement à 0,2 fois cette hauteur.

Avantageusement la tête motrice et la tête de refoulement appartiennent respectivement à deux pistons superposés. L'un, côté amont, sera appelé le piston supérieur ; l'autre côté aval, vers le réservoir de liquide, sera appelé piston inférieur. Ces pistons comportent des dispositifs d'étanchéité tels que par exemple des joints ou des lèvres qui permettent leurs déplacement dans des chambres cylindriques appropriées : chambre motrice pour la tête motrice et réservoir pour la tête de refoulement.

Avantageusement le rapport des sections de la tête motrice et de la tête de refoulement est supérieur ou égal à 1,1. Préférentiellement ce rapport est compris entre 1,2 et 8 et se situe avantageusement entre 2 et 6.

Le choix de la course motrice, par rapport à la hauteur du réservoir et le choix du rapport des sections des têtes motrice et de refoulement permet de régler, en niveau et en durée, les pressions et donc les conditions d'injection du principe actif liquide, notamment les vitesses d'injection.

Dans un première réalisation de la seringue selon l'invention la tête motrice est constituée par l'emboîtement d'un piston supérieur annulaire sur une tige amont solidaire d'un piston inférieur comportant la tête de refoulement. Cet emboîtement est un emboîtement cylindrique coulissant ou un emboîtement conique. Il est étanche. La pression des gaz s'exerce sur une grande surface : la résultante est grande. La pression transmise au liquide est forte et la vitesse d'injection est élevée.

Dans cette réalisation le dispositif limitant la course motrice de la tête motrice est une butée au fond aval du cylindre moteur. Cette butée arrête le piston supérieur annulaire. Les gaz agissent sur la tige amont du piston inférieur et la repoussent. Dans ce déplacement lorsque l'étanchéité entre le piston annulaire et la tige amont est rendue inopérante par le déplacement de ladite tige amont, la pression des gaz s'exerce sur une section égale à celle du réservoir, la résultante des forces de pression est plus faible que précédemment et la pression transmise au liquide est plus faible ainsi que la vitesse.

Dans une deuxième réalisation de la seringue la tête motrice est la face amont d'un piston supérieur plein et la tête de refoulement est la face aval d'un piston inférieur plein. Plus précisément, la face amont du piston supérieur de grande section sera la tête motrice. La face aval du piston inférieur, de section plus petite et égale à celle du réservoir, sera la tête de refoulement.

Dans une première variante de cette réalisation les deux pistons sont séparés par une distance D. Une protubérance intégrée à l'un des pistons permet de déterminer cette distance D. Le piston supérieur vient au contact du piston inférieur après s'être déplacé de cette distance D et il y a un effet de choc. Pour rester dans le cadre de l'invention cette distance D doit être inférieure à la course motrice de la tête motrice

Dans une deuxième variante les deux pistons sont en contact : c'est en fait le cas où la distance D est nulle.

Pour ces deux variantes le dispositif limitant la course motrice de la tête motrice est une chambre d'expansion prolongeant le cylindre moteur. Cette chambre d'expansion est réalisée par un élargissement du cylindre moteur, soit par une augmentation du diamètre, soit par un ensemble de rainures latérales qui vont laisser passer les gaz autour de la tête motrice et de son dispositif d'étanchéité de façon que le piston supérieur ne soit plus soumis à un effort résultant du fait de la pression et que les gaz agissent sur le piston inférieur plein sur une section égale à celle du réservoir.

Dans ces variantes, quand le piston supérieur plein comportant la tête motrice est dans la chambre d'expansion, il cesse d'être moteur et, en fin de course, il vient en appui sur une surface comportant des passages de gaz pour que les dits gaz agissent sur le piston inférieur.

Dans une troisième réalisation de la seringue sans aiguille selon l'invention, le moyen de poussée est un piston unique, étagé, dont la face amont appartient à la tête motrice de grande section et la face aval appartient à la tête de refoulement de plus faible section. C'est le cas particulier où les deux pistons précédemment cités sont solidaires.

Dans cette réalisation, le dispositif limitant la course motrice de la tête motrice est une chambre d'expansion prolongeant le cylindre moteur. Cette chambre d'expansion est réalisée de la même façon que précédemment par exemple par un élargissement du cylindre moteur soit par une augmentation du diamètre, soit par un ensemble de rainures longitudinales et latérales qui vont laisser passer le gaz autour de la tête motrice de façon que les gaz agissent sur une section équivalente à celle de la tête de refoulement. Dans cette réalisation la chambre d'expansion doit être suffisamment longue pour que le piston se déplace jusqu'à ce qu'il arrive en butée sur la face amont de l'injecteur pour faire l'injection de tout le liquide contenu dans le réservoir.

Dans cette invention le moyen de poussée, dans ses différentes réalisations, est déplacé par les gaz d'un générateur de gaz. Ces gaz pouvant être générés par une réaction chimique ou par détente brutale d'une réserve de gaz comprimé.

Mais préférentiellement le générateur de gaz est un générateur pyrotechnique. Ce type de générateur est préféré pour sa puissance, sa compacité et sa fiabilité, qui permet d'envisager pour des seringues pré-remplies, des durées de stockage seulement limitées par celles de la conservation du principe actif.

La présente invention résout le problème posé de pouvoir prédéterminer simplement les deux niveaux de vitesse d'injection ainsi que leur durée.

La présente invention a l'avantage de distinguer dans le dispositif deux parties. Une partie pharmaceutique comprenant le corps et le réservoir avec un injecteur à l'aval et un piston à l'amont : ce sous-ensemble pourra être traité dans les conditions de l'industrie pharmaceutique notamment en ce qui concerne la stérilisation et l'asepsie. Ce sous ensemble sera intégré au reste de la seringue, dont les éléments ont été assemblés par ailleurs, cet assemblage se faisant dans les conditions moins sévères que celles liées à l'industrie pharmaceutique.

Ci-dessus l'invention est exposée en détail à l'aide de figures représentant différentes réalisations particulières de l'invention.

La figure 1 représente une coupe longitudinale d'une seringue qui n'est pas partie de la présente invention. La figure 2 représente, en vue partielle, la position des pistons après fonctionnement.

Les figures 3 et 4 représentent, en vues partielles, respectivement les pistons en positions initiale puis finale pour une seringue, selon l'invention, comportant deux pistons pleins séparés.

La figure 5, représente en vue partielle une réalisation avec un piston étagé unique, en position initiale.

La figure 6, illustre un exemple de courbes de pression dans la chambre du générateur de gaz et dans le liquide.

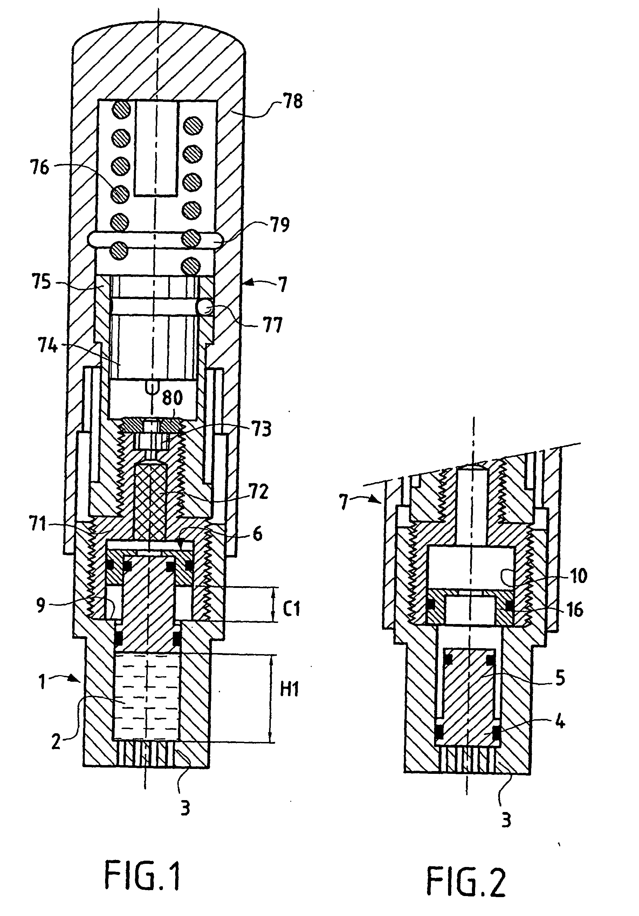

La figure 1 représente schématiquement, avant utilisation et, en coupe longitudinale partielle, une seringue sans aiguille qui n'est pas partie de la présente invention. La seringue est représentée verticale, l'injecteur à son extrémité aval dirigé vers le bas.

Cette seringue comporte, du haut vers le bas, un générateur de gaz 7 qui sera décrit plus en détail par la suite. Ce générateur de gaz est fixé par son corps 71 sur le réservoir 2 de la seringue. Un piston annulaire 16, qui est un élément de la tête motrice 6 est monté par un emboîtement cylindrique coulissant sur l'extrémité amont ou tige amont 5 d'un deuxième piston dont l'extrémité aval opposée est la tête de refoulement 4, l'appui du piston annulaire sur le piston de refoulement est assuré par un épaulement convenablement dimensionné. L'emboîtement des deux pistons est rendu étanche par au moins un joint torique ou tout autre moyen équivalent. Le piston annulaire 16 est de grande section vis à vis du piston portant la tête de refoulement. Ces pistons comportent des joints toriques, ou les moyens équivalents pour les rendre étanche vis à vis de leurs chambres de déplacement : le cylindre moteur 10 et le réservoir 2.

Nous allons décrire les principaux éléments, du générateur de gaz 7, qui dans cet exemple est pyrotechnique. Il comprend dans le corps 71, au dessus du piston comportant la tête motrice 6, un chargement pyrotechnique 72 dont la combustion est initiée par une amorce 73 impactée par un percuteur 74, ce percuteur n'est pas représenté en coupe mais vu de côté. L'amorce 73 est logée dans un porte-amorce. En position initiale le percuteur 74 est retenu, dans le guide-percuteur 75 solidaire par vissage du corps 71, par au moins une bille, telle que la bille 77, partiellement engagée dans une gorge du percuteur. Le dispositif de percussion comprend un poussoir 78 avec une gorge 79 et un ressort intérieur 76.

Le poussoir 78 coulisse sur l'extérieur du guide-percuteur 75 et il est retenu par des ergots se déplaçant dans des rainures latérales. Ce poussoir 78 est ici l'organe de déclenchement.

Bien entendu pour initier la combustion du chargement pyrotechnique 72, sans sortir du cadre de l'invention, on peut utiliser des dispositifs d'initiation autre que le dispositif à percuteur ici décrit. Sans entrer dans les détails et sans vouloir être exhaustif, nous citerons comme exemples des dispositifs d'initiation à pile électrique ou des dispositifs d'initiation piézo-électrique.

Eventuellement le générateur de gaz pyrotechnique peut être remplacé par un générateur de gaz constitué par un réservoir de gaz comprimé fermé par une vanne à ouverture rapide. L'organe de déclenchement va ouvrir ladite vanne, les gaz comprimés du réservoir vont se détendre et agir sur le moyen de poussée.

Sur cette figure 1 la seringue est prête à l'emploi, quand l'injecteur est en appui sur la peau du sujet à traiter (non représentée). L'opérateur appui, avec son pouce, sur le poussoir 78 qui s'enfonce en comprimant le ressort 76. Le poussoir se déplace jusqu'à ce que la gorge 79 arrive à la hauteur de la gorge du percuteur 74, les billes, telle que la bille 77, retenant le percuteur 74, se dégagent dans la gorge 79 et libèrent le percuteur qui va impacter violemment l'amorce 73, dont l'initiation enflamme le chargement pyrotechnique 72. Le percuteur en appui sur le porte-amorce 80 assure l'étanchéité : les gaz de combustion ne remontant pas vers le poussoir 78.

Au cours d'une première phase de fonctionnement les gaz du générateur 7 agissent sur la grande surface amont du piston annulaire 16 dont l'ouverture centrale est fermée de façon étanche par l'extrémité amont de la tige 5 du piston portant la tête de refoulement 4. Le long de la course C₁ les deux pistons se déplacent ensemble, la force résultante s'exerçant sur le moyen de poussée est grande, la pression transmise au liquide par la tête de refoulement 4 est grande : l'effet multiplicateur sur la pression est en première approximation lié au rapport de sections. Le principe actif liquide va sortir des conduits de l'injecteur 3 avec une grande vitesse et percer efficacement et pénétrer dans la peau du sujet à traiter. La course motrice C₁ de la tête motrice 6 dans son cylindre moteur 10 est inférieure à la hauteur H₁ du réservoir.

Ensuite au cours du déplacement de l'ensemble des deux pistons, le piston annulaire de grande section arrive en butée sur le fond 9 de la chambre formée par le cylindre moteur 10, le piston annulaire est bloqué sur ce fond 9. Les gaz agissent sur l'extrémité amont 5 de l'autre piston qui va continuer à se déplacer sous l'effet d'une force réduite jusqu'à ce que le joint n'assure plus l'étanchéité, alors la section efficace du piston comportant la tête de refoulement 4 est égale à la section du réservoir 2, section plus petite que la section efficace de la première phase. La pression d'injection est plus faible ainsi que la vitesse du jet de liquide.

Pour réduire au minimum la phase transitoire lors du déboîtement des deux pistons il est préférable d'une part que la section de la partie amont 5 soit très voisine de celle de la tête de refoulement et d'autre part que l'ouverture du piston annulaire soit la plus grande possible tout en assurant un épaulement et un appui convenable du piston annulaire sur la partie amont ; sur cette figure, pour plus de clarté les écarts ont été accentués.

Cette réduction de phase transitoire peut également être obtenue en utilisant un emboîtement avec une portée conique entre le piston annulaire 16 et la tige amont du piston de refoulement. Eventuellement un évent, percé à travers la partie aval 9 et le corps 71, pour évacuer l'air compris entre le piston annulaire 16 et la tige amont 5 du piston de refoulement, contribue à la réduction de l'importance de la phase transitoire.

La figure 2 représente, en coupe partielle, la partie aval de la seringue en fin d'injection. Le piston annulaire 16 est en butée sur le fond 9 de sa chambre motrice. L'autre piston, comportant la tête de refoulement s'est déplacé jusqu'à arriver en butée sur la face intérieure amont de l'injecteur 3.

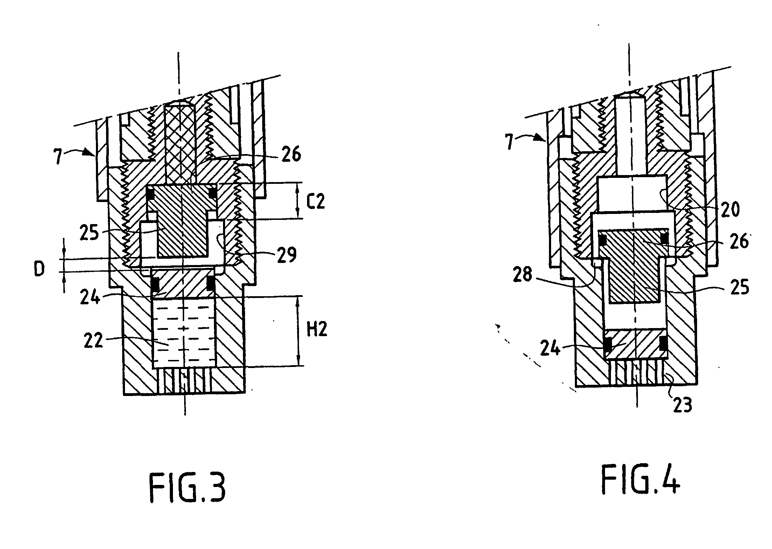

Une première réalisation de l'invention est représentée sur les figures 3 et 4. Sur ces figures sont représentés respectivement l'état initial et l'état final de la partie aval de la seringue. Le moteur est du type de celui décrit, à titre d'exemple, sur la figure 1, il continuera à être désigné par le repère 7.

La figure 3 représente la configuration initiale de cette réalisation. Le piston portant la tête motrice 26 de grande section est un piston plein, il coulisse dans une chambre motrice 20. La face aval du piston portant la tête motrice comporte une protubérance 25 séparée par une distance D de la face amont du piston portant la tête de refoulement 24 qui ferme le réservoir de liquide. Lorsque le fonctionnement du générateur de gaz est initié, les gaz repoussent la tête motrice 26 avec une grande force (pression s'exerçant sur une grande surface), le piston moteur 26 après avoir parcouru la distance D impacte le piston de refoulement 24 et lui transmet cette grande force jusqu'à ce que le piston moteur 26 se dégage dans la chambre d'expansion 29. La course motrice C₂ de la tête motrice 26 dans son cylindre moteur 20 est inférieure à la hauteur H₂ du réservoir. Cette chambre d'expansion est dans cet exemple une chambre de diamètre supérieur à celui de la section motrice 20 les gaz peuvent circuler autour de la tête motrice 26 de grande section, l'effort résultant sur les pistons 26 et 24 est celui correspondant à la pression des gaz s'exerçant sur une surface égale à la section du piston portant la tête de refoulement 24. Cet effort est plus faible et la pression dans le liquide est plus faible. Le piston de refoulement 24 continue son déplacement jusqu'à ce que tout le liquide soit éjecté du réservoir. Le piston moteur 26 est arrêté, sur une surface 28 permettant le passage des gaz pour que ceux-ci agissent sur le piston de refoulement. La surface 28 est par exemple un ensemble de nervures dans la partie aval de la chambre 29.

La figure 4 représente le dispositif dans son état final. Le tête motrice 26 s'est dégagée dans la chambre d'expansion 29, elle est arrêtée sur la surface 28 permettant le passage des gaz. Le piston de refoulement 24 poursuivi son déplacement jusqu'à ce qu'il arrive en butée sur la face amont de l'injecteur 23.

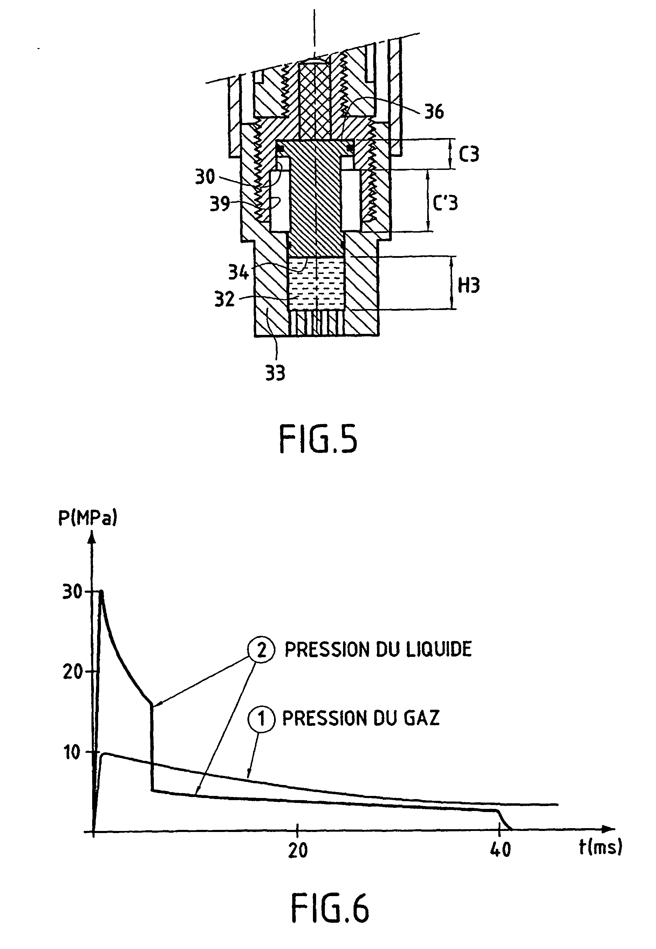

Enfin une deuxième réalisation de l'invention est représentée figure 5. Elle représente la partie aval de la seringue avec un piston unique en position initiale.

Dans cette réalisation le moyen de poussée est un piston étagé unique : son extrémité de grande section ou tête motrice 36 est tournée vers le générateur de gaz 7 ; son extrémité de petite section ou tête de refoulement 34 est en appui sur le principe actif . Sur une course C₃ l'extrémité de la tête motrice 36 se déplace dans un cylindre moteur 30 de grande section, donc l'effort efficace résultant est grand et la pression transmise au liquide est forte. Cette course motrice C3 est inférieure à la hauteur H₃ du réservoir. Puis cette extrémité 36 se dégage dans une chambre d'expansion 39 dont la section est supérieure à celle du cylindre moteur 30. Comme précédemment cette section supérieure est réalisée par un diamètre de la chambre 39 supérieur à celui du cylindre moteur 30. Les gaz pouvant circuler autour de l'extrémité 36, la résultante de la pression sera celle s'exerçant sur une section égale à celle du réservoir et de la tête de refoulement 34 : la pression transmise au liquide sera plus faible. La longueur C'₃ de la chambre d'expansion sera telle que le piston poursuit son déplacement jusqu'à ce que la tête de refoulement 34 arrive en butée sur la face amont de l'injecteur 33.

Pour une seringue du type de celle représentée sur la figure 1 et qui a été équipée de capteurs de pression la figure 6 représente, en fonction du temps, l'évolution de la pression du gaz côté générateur (courbe 1) et celle de la pression du liquide (courbe 2) côté réservoir. Le générateur de gaz est du type pyrotechnique, il comporte une charge de poudre à base de nitrocellulose. Le rapport des sections des têtes motrice et de refoulement est de 3,1.

En début d'injection sous l'effet de la pression transmise par le piston à tête motrice la pression d'injection du liquide est près de trois fois celle du gaz. La transition d'un régime à l'autre est très rapide, l'instrumentation ne permet pas de la suivre de façon détaillée. Pour le deuxième régime la pression dans le liquide suit celle des gaz, aux frottements près.

## Revendications

1. Seringue sans aiguille (21, 31) pour l'injection d'un principe actif liquide contenu dans un réservoir (22, 32) compris entre d'une part un injecteur (23, 33) comportant au moins un conduit d'injection et d'autre part un moyen de poussée soumis à l'action d'un générateur de gaz (7), ledit moyen de poussée comportant une première extrémité, dirigée vers le principe actif, qui constitue une tête de refoulement (24, 34) monobloc dont la section transversale est égale à la section du réservoir, et une deuxième extrémité qui est dirigée vers le générateur de gaz (7) et présentant une tête motrice (26, 36) dont la section transversale est supérieure à la section de la tête de refoulement, la course motrice (C₂, C₃) de la tête motrice dans son cylindre moteur (20, 30) étant inférieure à la hauteur (H₂, H₃) du réservoir (22, 32) de principe actif, **caractérisée en ce que** le dispositif limitant la course motrice (C₂, C₃) de la tête motrice (26, 36), est une chambre d'expansion (29, 39) prolongeant le cylindre moteur (20, 30).

2. Seringue sans aiguille selon la revendication 1 **caractérisée en ce que** la tête motrice (26) est la face amont d'un piston supérieur plein et que la tête de refoulement (24) est la face aval d'un piston inférieur plein.

3. Seringue sans aiguille selon la revendication 2 **caractérisée en ce que** les deux pistons sont séparées par une distance (D).

4. Seringue sans aiguille selon la revendication 2 **caractérisée en ce que** les deux pistons sont en contact.

5. Seringue sans aiguille selon la revendication 2 **caractérisée en ce que** le piston supérieur plein comportant la tête motrice (26) vient en appui sur une surface (28) comportant des passages de gaz.

6. Seringue sans aiguille selon la revendication 1 **caractérisée en ce que** le moyen de poussée est un piston unique, étagé, dont la face amont appartient à la tête motrice (36) et la face aval est la tête de refoulement (34).

7. Seringue sans aiguille selon la revendication 6 **caractérisée en ce que** la largeur de la chambre d'expansion (29) permet le contact entre la tête de refoulement (34) et la face amont de l'injecteur.

8. Seringue sans aiguille selon la revendication 1 **caractérisée en ce que** le générateur de gaz (7) est un générateur pyrotechnique.

## Patentansprüche

1. Nadellose Spritze (21, 31) zum Spritzen eines flüssigen Wirkstoffs, der in einem Reservoir (22, 32) enthalten ist, das sich zwischen einerseits einem Injektor (23, 33), der mindestens einen Injektionskanal aufweist, und andererseits einem Schubmittel befindet, das der Einwirkung eines Gasgenerators (7) unterworfen ist, wobei das Schubmittel ein erstes, zum Wirkstoff gerichtetes Ende, das einen einstückigen Förderkopf (24, 34) bildet, dessen Querschnitt gleich dem Querschnitt des Reservoirs ist, und ein zweites, zum Gasgenerator (7) gerichtetes Ende aufweist, das einen Antriebskopf (26, 36) besitzt, dessen Querschnitt größer ist als der Querschnitt des Förderkopfs, wobei der Antriebshub (C₂, C₃) des Antriebskopfs in seinem Antriebszylinder (20, 30) kürzer ist als die Höhe (H₂, H₃) des Wirkstoffreservoirs (22, 32), **dadurch gekennzeichnet, dass** die den Antriebshub (C₂, C₃) des Antriebskopfs (26, 36) begrenzende Vorrichtung eine Ausdehnungskammer (29, 39) ist, die den Antriebszylinder (20, 30) verlängert.

2. Nadellose Spritze nach Anspruch 1, **dadurch gekennzeichnet, dass** der Antriebskopf (26) die Vorderseite eines oberen massiven Kolbens ist, und dass der Förderkopf (24) die Rückseite eines unteren massiven Kolbens ist.

3. Nadellose Spritze nach Anspruch 2, **dadurch gekennzeichnet, dass** die beiden Kolben durch eine Entfernung (D) voneinander entfernt sind.

4. Nadellose Spritze nach Anspruch 2, **dadurch gekennzeichnet, dass** die beiden Kolben in Kontakt sind.

5. Nadellose Spritze nach Anspruch 2, **dadurch gekennzeichnet, dass** der den Antriebskopf (26) enthaltende obere massive Kolben auf einer Fläche (28) in Anlage kommt, die Gasdurchlässe aufweist.

6. Nadellose Spritze nach Anspruch 1, **dadurch gekennzeichnet, dass** das Schubmittel ein einziger gestufter Kolben ist, dessen Vorderseite zum Antriebskopf (36) gehört und dessen Rückseite der Förderkopf (34) ist.

7. Nadellose Spritze nach Anspruch 6, **dadurch gekennzeichnet, dass** die Breite der Ausdehnungskammer (29) den Kontakt zwischen dem Förderkopf (34) und der Vorderseite des Injektors erlaubt.

8. Nadellose Spritze nach Anspruch 1, **dadurch gekennzeichnet, dass** der Gasgenerator (7) ein pyrotechnischer Generator ist.

## Claims

1. Needle-less syringe (21, 31) for injecting a liquid active principle contained in a reservoir (22, 32) included between, on the one hand, an injector (23, 23) having at least one injection conduit and, on the other hand, a thrust means which is subjected to the action of a gas-generator (7), the said thrust means having a first end which is directed towards the active principle and constitutes a one-piece discharging head (24, 34) whose cross-section is equal to the section of the reservoir, and a second end which is directed towards the gas-generator (7) and has a driving head (26, 36) whose cross-section is larger than the section of the discharging head, the driving travel (C₂, C₃) of the driving head within its driving cylinder (20, 30) being lower than the height (H₂, H₃) of the active principle reservoir (22, 32), **characterised in that** the device which limits the driving travel (C₂, C₃) of the driving head (26, 36) is an expansion chamber (29, 39) which prolongs the driving cylinder (20, 30).

2. Needle-less syringe according to claim 1, **characterised in that** the driving head (26) is the upstream face of a solid upper piston, and that the discharging head (24) is the downstream face of a solid lower piston.

3. Needle-less syringe according to claim 2, **characterised in that** the two pistons are separated by a distance (D).

4. Needle-less syringe according to claim 2, **characterised in that** the two pistons are in contact.

5. Needle-less syringe according to claim 2, **characterised in that** the solid upper piston having the driving head (26) rests on a surface (28) having gas passages.

6. Needle-less syringe according to claim 1, **characterised in that** the thrust means is a single, tiered piston whose upstream face belongs to the driving head (36) and whose downstream face is the discharging head (34).

7. Needle-less syringe according to claim 6, **characterised in that** the width of the expansion chamber (29) permits contact between the discharging head (34) and the upstream face of the injector.

8. Needle-less syringe according to claim 1, **characterised in that** the gas-generator (7) is a pyrotechnical generator.
